(19) Europäisches Patentamt
European Patent Office
Office européen des brevets

(11) EP 1 466 900 B1

(12) **EUROPEAN PATENT SPECIFICATION**

(45) Date of publication and mention of the grant of the patent:
**14.01.2009 Bulletin 2009/03**

(21) Application number: **02793377.9**

(22) Date of filing: **25.12.2002**

(51) Int Cl.:
*C07C 401/00* (2006.01) *A61K 31/59* (2006.01)
*A61P 3/02* (2006.01) *A61P 19/08* (2006.01)
*A61P 35/00* (2006.01) *A61P 35/02* (2006.01)

(86) International application number:
**PCT/JP2002/013505**

(87) International publication number:
**WO 2003/055854 (10.07.2003 Gazette 2003/28)**

(54) **2-SUBSTITUTED VITAMIN D DERIVATIVES**

2-SUBSTITUIERTE VITAMIN-D-DERIVATE

DERIVES DE LA VITAMINE D-DOUBLE SUBSTITUTION

(84) Designated Contracting States:
**AT BE BG CH CY CZ DE DK EE ES FI FR GB GR IE IT LI LU MC NL PT SE SI SK TR**

(30) Priority: **26.12.2001 JP 2001393881**

(43) Date of publication of application:
**13.10.2004 Bulletin 2004/42**

(73) Proprietor: **Takayama, Hiroaki**
**Tokyo 151-0072 (JP)**

(72) Inventors:
- **TAKAYAMA, Hiroaki**
  **Shibuya-ku, Tokyo 151-0072 (JP)**
- **FUJISHIMA, Toshie**
  **Hachioji-shi, Tokyo 193-0834 (JP)**
- **KITTAKA, Atsushi**
  **Kunitachi-shi, Tokyo 186-0002 (JP)**

(74) Representative: **Vossius & Partner**
**Siebertstraße 4**
**81675 München (DE)**

(56) References cited:
**WO-A1-01/90061**

- **KONNO KATSUHIRO ET AL: "Synthesis, biological evaluation, and conformational analysis of A-ring diastereomers of 2-methyl-1,25-dihydroxyvitamin D3 and their 20-epimers: Unique activity profiles depending on the stereochemistry of the A-ring and at C-20" JOURNAL OF MEDICINAL CHEMISTRY, vol. 43, no. 22, 2 November 2000 (2000-11-02), pages 4247-4265, XP002328807 ISSN: 0022-2623**
- **SUHARA Y ET AL: "Syntheses and biological evaluation of novel 2alpha-substituted 1alpha,25-dihydroxyvitamin D3 analogues" BIOORGANIC & MEDICINAL CHEMISTRY LETTERS, OXFORD, GB, vol. 10, no. 10, May 2000 (2000-05), pages 1129-1132, XP004204623 ISSN: 0960-894X**
- **FUJISHIMA T ET AL: "Highly potent cell differentiation-inducing analogues of 1[alpha], 25-dihydroxyvitamin D3: Synthesis and biological activity of 2-methyl-1,25-dihydroxyvitamin D3 with side-chain modifications" BIOORGANIC AND MEDICINAL CHEMISTRY 2001 UNITED KINGDOM, vol. 9, no. 2, February 2001 (2001-02), pages 525-535, XP002328808 ISSN: 0968-0896**
- **POSNER G H ET AL: "2,2-disubstituted analogues of the natural hormone 1alpha,25-dihydroxyvitamin D3: Chemistry and biology" BIOORGANIC & MEDICINAL CHEMISTRY, ELSEVIER SCIENCE LTD, GB, vol. 10, no. 7, July 2002 (2002-07), pages 2353-2365, XP002291417 ISSN: 0968-0896**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **FUJISHIMA TOSHIE ET AL: "Synthesis of 2,2-dimethyl-1,25-dihydroxyvitamin D3: A-ring structural motif that modulates interactions of vitamin D receptor with transcriptional coactivators." ORGANIC & BIOMOLECULAR CHEMISTRY. 7 JUN 2003, vol. 1, no. 11, 7 June 2003 (2003-06-07), pages 1863-1869, XP002328809 ISSN: 1477-0520**
- **FUJISHIMA TOSHIE ET AL.: 'Synthesis and biological evaluation of all A-ring stereoisomers of 5,6-trans-2-methyl-1,25-dihydroxyvitamin D3 and their 20-epimers: possible binding modes of potent A-ring analogues to vitamin D receptor' CHEMISTRY & BIOLOGY vol. 8, no. 11, 2001, pages 1011 - 1024, XP002965794**
- **SUHARA YOSHITOMO ET AL.: 'Efficient and versatile synthesis of novel 2alpha-substituted 1alpha,25-dihydroxyvitamin D3 analogues and their docking to vitamin D receptors' JOURNAL OF ORGANIC CHEMISTRY vol. 66, no. 26, 2001, pages 8760 - 8771, XP002965795**

## Description

### TECHNICAL FIELD

The present invention relates to novel vitamin D derivatives, and more particularly, to vitamin D derivatives having two substituents at the 2-position thereof.

### BACKGROUND ART

Active vitamin $D_3$ compounds, including 1α, 25-dihydroxyvitamin $D_3$, are known to have many physiological activities, such as tumor cell growth suppressing action, tumor cell differentiation inducing action, and immunomodulating action, as well as calcium metabolism regulating action. However, some active vitamins $D_3$ disadvantageously may cause hypercalcemia during long-term and continuous administration. Such compounds have been difficult to use as antitumor agents or antirheumatic agents. Thus, study is under way on the synthesis of numerous vitamin D derivatives, with the aim of separating the actions of these vitamin D compounds.

Studies by the present inventors have shown that the introduction of a 2α-methyl group into the A-ring portion of active vitamin $D_3$ (i.e., 1α,25-dihydroxyvitamin $D_3$) results in an increased ability to bind to vitamin D receptor (VDR) (K. Konno et al., Bioorg. Med. Chem. Lett., 1998, 8, 151). Furthermore, a combination of the introduction of the 2α-methyl group and the 20-epimerization of the side chain moiety has been reported to increase VDR-binding ability additively (T. Fujishima et al., Bioorg. Med. Chem. Lett., 1998, 8, 2145). Moreover, vitamin D derivatives having a 4-hydroxybutyl group or an acyloxy group at the 2α-position are known as vitamin D derivatives having a substituent at the 2α-position (J. Org. Chem., Vol. 59, No. 25, 1994 and Japanese Patent Application Laid-Open No. 1976-19752).

However, no reports have been issued on the synthesis of vitamin D derivatives having a plurality of substituents introduced at the 2-position. Nor have the physiological activities of such vitamin D derivatives been studied.

### DISCLOSURE OF THE INVENTION

In an attempt to provide vitamin D derivatives improved in the above-described points, we have focused on vitamin D derivatives having a plurality of substituents at the 2-position.

We conducted in-depth studies to solve the above task, and found that the intended object could be attained by vitamin D derivatives having two substituents at the 2-position, thereby accomplishing the present invention.

That is, according to the present invention, there are provided vitamin D derivatives represented by the following general formula (1):

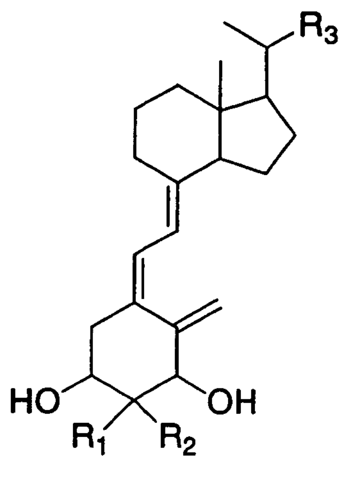

wherein $R_1$ and $R_2$ may be the same or different, and each represents a straight chain or branched chain alkyl group optionally substituted by a hydroxyl group, and $R_3$ represents a straight chain or branched chain alkyl group optionally substituted by a hydroxyl group.

In the general formula (1), it is preferred that $R_1$ and $R_2$ may be the same or different, and each represents a straight chain or branched chain alkyl group having 1 to 6 carbon atoms and optionally substituted by a hydroxyl group, and $R_3$ represents a straight chain or branched chain alkyl group having 1 to 12 carbon atoms and substituted by a

hydroxyl group.

More preferably, $R_1$ and $R_2$ may be the same or different, and each represents a straight chain or branched chain alkyl group having 1 to 3 carbon atoms and optionally substituted by a hydroxyl group, and $R_3$ represents a straight chain or branched chain alkyl group having 3 to 10 carbon atoms and substituted by a hydroxyl group.

Even more preferably, $R_1$ represents a methyl group, $R_2$ represents a methyl group, and $R_3$ represents a 4-hydroxy-4-methylpentyl group.

In the general formula (1), the steric configuration of the 20-position may be the S-configuration or the R-configuration.

Moreover, according to another aspect of the present invention, a pharmaceutical composition containing any of the above-described vitamin D derivatives is provided.

## PREFERRED MODES FOR CARRYING OUT THE INVENTION

The entire disclosure of Japanese Patent Application No. 2001-393881, the application on which the priority claim of the present application is based, is incorporated herein by reference in its entirety.

Detailed mode and specific examples for carrying out the vitamin D derivatives of Formula (1) of the present invention will be explained below.

Herein, a straight chain or branched chain alkyl group having 1 to 15 carbon atoms is preferred as the straight chain or branched chain alkyl group. Examples of such an alkyl group are, but not limited to, a methyl group, an ethyl group, an n-propyl group, an i-propyl group, an n-butyl group, an s-butyl group, an i-butyl group, a t-butyl group, and straight chain and branched chain alkyl groups such as a pentyl group, a hexyl group, a heptyl group, an octyl group, a nonyl group, and a decanyl group.

The straight chain or branched chain alkyl group optionally substituted by a hydroxyl group refers to the above-mentioned alkyl group in which arbitrary hydrogen atoms may be substituted by one or more hydroxyl groups.

The alkyl group as "the straight chain or branched chain alkyl group optionally substituted by a hydroxyl group" in the definitions of $R_1$ and $R_2$ is one preferably having 1 to 8 carbon atoms, more preferably 1 to 6 carbon atoms, and even more preferably 1 to 3 carbon atoms. Examples of the alkyl group are a methyl group, an ethyl group, an n-propyl group, an i-propyl group, an n-butyl group, an s-butyl group, an i-butyl group, a t-butyl group, a pentyl group, and a hexyl group.

Non-restrictive examples of $R_1$ and $R_2$ are a methyl group, a hydroxymethyl group, a hydroxyethyl group, a propyl group, a hydroxypropyl group, a butyl group, a hydroxybutyl group, a pentyl group, a hydroxypentyl group, a hexyl group, a hydroxyhexyl group, a heptyl group, a hydroxyheptyl group, an octyl group, a hydroxyoctyl group, a nonyl group, a hydroxynonyl group, a decanyl group, and a hydroxydecanyl group. Of these, a methyl group, an ethyl group, a hydroxymethyl group, a hydroxyethyl group, a hydroxypropyl group, or a hydroxybutyl group is preferred, and the most preferred is a methyl group.

The alkyl group as "the straight chain or branched chain alkyl group optionally substituted by a hydroxyl group" in the definition of $R_3$ is preferably one having 1 to 15 carbon atoms, more preferably 1 to 12 carbon atoms, even more preferably 3 to 10 carbon atoms, and further preferably 4 to 7 carbon atoms. Examples of the alkyl group are, but not limited to, a methyl group, an ethyl group, an n-propyl group, an i-propyl group, an n-butyl group, an s-butyl group, an i-butyl group, a t-butyl group, a pentyl group, a hexyl group, a heptyl group, an octyl group, a nonyl group, and a decanyl group. Any of these alkyl groups is preferably substituted by a hydroxyl group.

Non-restrictive examples of $R_3$ are a 4-hydroxy-4-methylpentyl group, a 4-ethyl-4-hydroxyhexyl group, a 6-hydroxy-6-methyl-2-heptyl group, a 7-hydroxy-7-methyl-2-octyl group, a 5,6-dihydroxy-6-methyl-2-heptyl group, and a 4,6,7-trihydroxy-6-methyl-2-heptyl group. Preferably, $R_3$ is a 4-hydroxy-4-methylpentyl group.

The vitamin D derivatives represented by the general formula (1) according to the present invention can be used as active ingredients for pharmaceutical compositions (for example, calcium metabolism regulators).

The vitamin D derivatives represented by the general formula (1) according to the present invention are novel compounds, and methods for synthesizing them are not limited. For example, the vitamin D derivatives of the present invention can be synthesized from hydroxy esters which are known compounds.

For example, when commercially available methyl hydroxypivalate (Tokyo Kasei or the like) is used as a starting material, the hydroxyl group is protected to form a p-methoxyphenyl ether-protected compound. This protected compound is reduced with a reducing agent, such as lithium aluminum hydride, to form an alcohol whose PDC oxidation furnishes an aldehyde. This aldehyde is reacted with an organometallic reagent, such as allenylmagnesium bromide, to obtain an acetylene derivative. The secondary hydroxyl group of the acetylene derivative is silylated, and subsequent deprotection of the protective group on the primary hydroxyl group furnishes an alcohol. This alcohol is converted into an aldehyde by PDC oxidation or the like, and the aldehyde is reacted with an organometallic reagent, such as vinylmagnesium bromide, to form enyne compounds. The resulting mixture of enyne compounds is separated into a 1,3-syn compound with the substituents at the 1-position and the 3-position configured as $1\alpha,3\alpha$ or $1\beta,3\beta$, and a 1,3-anti compound with

the substituents at the 1-position and the 3-position configured as 1α,3β or 1β,3α, by a conventional method such as silica gel column chromatography. Then, the secondary hydroxyl groups of the respective enyne compounds are silylated to obtain A-ring precursors.

Reaction of the respective A-ring precursors with CD-ring bromoolefin in a suitable solvent with the use of palladium results in the construction of 2,2-substituted vitamin D skeletons. The resulting protected compounds are subjected to a deprotection step, and purified by a conventional method, such as reversed-phase HPLC or thin-layer chromatography to obtain the desired vitamin D derivatives. Alternatively, the protected compounds may be purified and then subjected to deprotection.

As the compounds serving as the CD-ring portion of the vitamin D derivatives, known compounds can be used. Alternatively, the desired CD-ring compounds can be obtained by starting with known CD-ring compounds, and modifying the side chains as appropriate. As another alternative, the CD-ring compounds can also be obtained from known vitamin D derivatives having corresponding side chains.

In using the compounds of the present invention as medicaments, it is preferred to use them after formulating them into suitable dosage forms in combination with pharmaceutically acceptable carriers, excipients, disintegrants, lubricants, binders, flavors, and colorants. Examples of such dosage forms are tablets, granules, fine granules, capsules, powders, injections, solutions, suspensions, emulsions, preparations for percutaneous absorption, and suppositories.

The route of administration of the compounds according to the present invention as pharmaceutical products is not limited, and they may be administered orally or parenterally (e.g., intravenously, intramuscularly, intraperitoneally, or percutaneously).

The dose of the compounds according to the present invention as pharmaceutical products can be selected, as appropriate, depending on the disease to be dealt with, the condition of the patient, the patient's physique, constitution, age or sex, the route of administration, the dosage form, and so on. Generally, the lower limit of the dose is in the range of 0.001 μg to 0.1 μg, preferably about 0.01 μg, per adult per day. The upper limit of the dose can be selected in the range of 100 μg to 10,000 μg, preferably 200 μg to 1,000 μg, per adult per day. This dose can be administered as a single daily dose or as two or three divided doses per day.

The present invention will now be described more concretely by the following examples, but is in no way limited by these examples:

Examples

(Example 1): Synthesis of compounds corresponding to the side chain moiety of vitamin D derivatives

In the following examples, the abbreviations shown below were used.

THF: Tetrahydrofuran
DEAD: Diethyl azodicarboxylate
EA: Ethyl acetate
PDC: Pyridinium dichromate
TBAF: Tetrabutylammonium fluoride
TBSOTf: tert-Butyldimethylsilyl triflate
CAN: Ceric ammonium nitrate

Commercially available reagents were used as they were, unless otherwise specified.

Merck Silica Gel 60 was used for silica gel column chromatography, and Merck Silica Gel 5744 was used for silica gel thin-layer chromatography.

Recycling reversed-phase HPLC was performed at a flow rate of 9.9 mL/min on a YMC-pack ODS column (20x150 mm) by means of a Waters 510 HPLC pump. Detection was performed using a Waters 484 tunable absorbance detector.

NMR spectra were measured with the use of JEOL GSX-400 or JEOL ECP-600.

Mass spectra were measured by the EI method using JEOL JMS-SX 102A.

Synthesis was carried out in accordance with the following reaction schemes:

Scheme 1

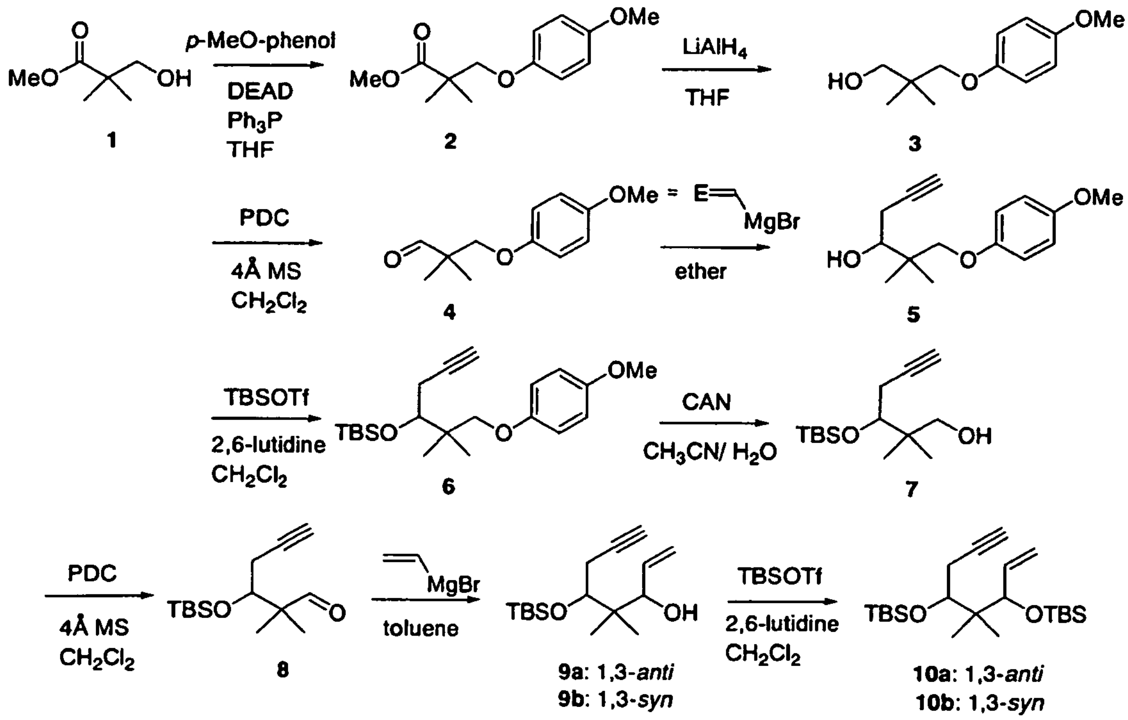

p-MeO-phenol
DEAD
$Ph_3P$
THF
1
2
$LiAlH_4$
THF
3
PDC
4Å MS
$CH_2Cl_2$
4
= E
MgBr
ether
5
TBSOTf
2,6-lutidine
$CH_2Cl_2$
6
CAN
$CH_3CN/ H_2O$
7
PDC
4Å MS
$CH_2Cl_2$
8
MgBr
toluene
**9a**: 1,3-*anti*
**9b**: 1,3-*syn*
TBSOTf
2,6-lutidine
$CH_2Cl_2$
**10a**: 1,3-*anti*
**10b**: 1,3-*syn*

Scheme 2

10a: 1,3-*anti*

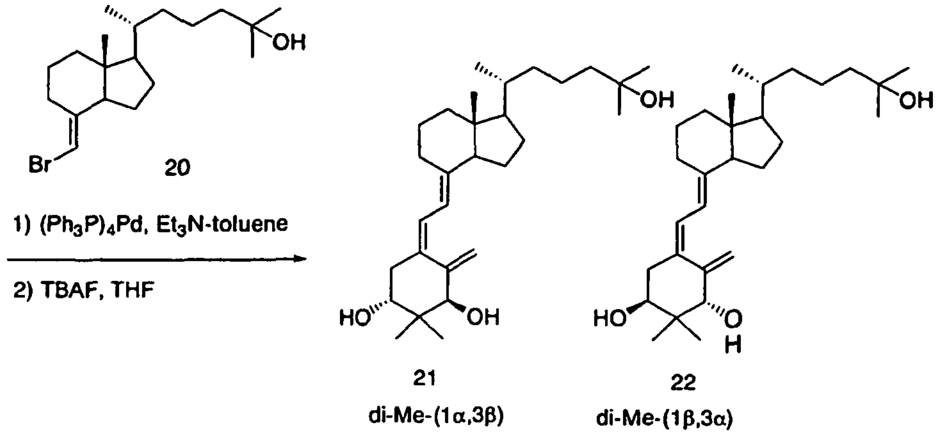


20

21 di-Me-(1α,3β)

22 di-Me-(1β,3α)

Scheme 3

10b: 1,3-*syn*

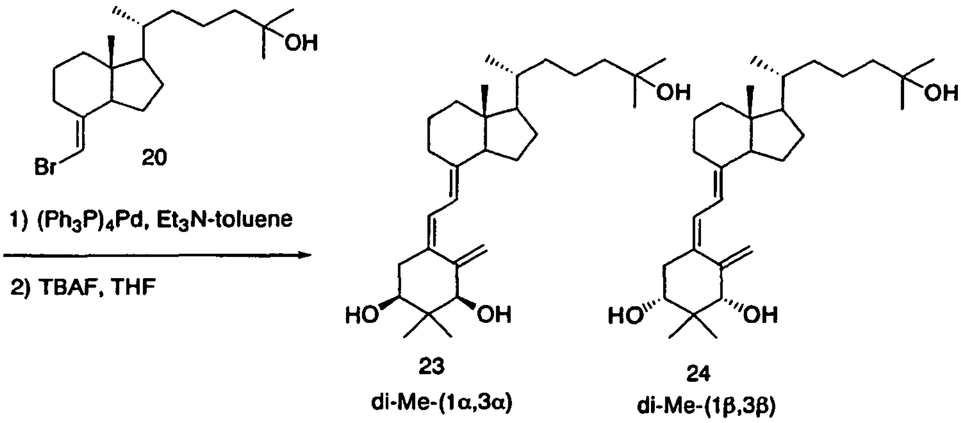


20

23 di-Me-(1α,3α)

24 di-Me-(1β,3β)

(Example 1) Synthesis of methyl 3-(4-methoxyphenoxy)-2,2-dimethylpropionate (Compound 2)

Methyl hydroxypivalate (Compound 1) (3.00 g, 22.7 mmols), p-methoxyphenol (8.45 g, 3 eq (equivalents)), and triphenylphosphine (7.74 g, 1.3 eq) were dissolved in dry THF (50 ml), and a 40% DEAD solution (13 mL, 1.3 eq) in toluene was added dropwise at 0°C. Under an argon atmosphere, the resulting mixture was refluxed for 2 hours, and then the solvent was distilled off. The residue was purified by silica gel column chromatography (EA:n-hexane = 1:9) to afford the captioned compound as a colorless oil (5.30 g, yield 98%).

Compound 2: $^1H$ NMR (400 MHz/$CDCl_3$/TMS) δ 1.30 (6 H, s), 3.69 (3 H, s), 3.76 (3 H, s), 3.91 (2 H, s), 6.82 (4 H, m). MS 238 (M+), 207 (M-OMe)+.
HRMS calcd. for $C_{13}H_{18}O_4$: 238.1205, found: 238.1206.

(Example 2) Synthesis of 3-(4-methoxyphenoxy)-2,2-dimethylpropanol (Compound 3)

A THF solution (15 mL) of Compound 2 (2.07 g, 8.39 mmols), which was an ester, was added dropwise to a THF suspension (10 mL) of $LiAlH_4$ (478 mg, 1.5 eq) at 0°C. After a lapse of 1.5 hours, EA and water were added to the resulting reaction mixture, and the system was filtered over CELITE (trade mark). The filtrate was extracted with EA. The resulting EA layer was dried over $MaSO_4$, and further filtered. The solvent was distilled off from the filtrate, and the residue was purified by silica gel column chromatography (EA:n-hexane = 1:3) to obtain the captioned compound as colorless crystals (1.71 g, yield 97%).

Compound 3: $^1H$ NMR (400 MHz/$CDCl_3$/TMS) δ 1.02 (6 H, s), 2.01 (1 H, brs), 3.54 (2 H, m), 3.73 (2 H, s), 3.77 (3 H, s), 6.83 (4 H, m).
MS 210 (M+).

HRMS calcd. for $C_{12}H_{18}O_3$: 210.1256, found: 210.1265.

(Example 3) Synthesis of 3-(4-methoxyphenoxy)-2,2-dimethylpropanal (Compound 4)

4Å Molecular sieve (500 mg) was added to a $CH_2Cl_2$ solution (20 mL) of Compound 3 (1.67 g, 7.94 mmols), which was an alcohol, and PDC (7.45 g, 2.5 eq) was added at 0°C under an argon atmosphere. The resulting mixture was left to stand overnight at room temperature. The resulting reaction product was purified by silica gel column chromatography (EA:n-hexane = 1:3) to obtain the captioned compound as a colorless oil (1.47 g, yield 89%).

Compound 4: $^1$H NMR (600 MHz/$CDCl_3$/TMS) δ 1.20 (6 H, s), 3.77 (3 H, s), 3.91 (2 H, s), 6.82 (4 H, s), 9.64 (1 H,s). MS 208 (M+).
HRMS calcd. for $C_{12}H_{16}O_3$: 208.1099, found: 208.1079.

(Example 4) Synthesis of 1-(4-methoxyphenoxy)-2,2-dimethylhex-5-yn-3-ol (Compound 5)

An allenylmagnesium bromide solution (ca. 2M, 66 mL, 3 eq) in ether was added dropwise to an ether solution of Compound 4 (4.73 g, 22 mmols), which was an aldehyde, at -78°C under an argon atmosphere, and the mixture was stirred for 90 minutes at -78°C. A saturated $NH_4Cl$ solution was added to the resulting mixture, and the system was extracted with EA. The EA layer was washed with brine, dried over $MaSO_4$, and filtered. Then, the solvent was distilled off, and the residue was purified by silica gel column chromatography (EA:n-hexane = 1:9) to obtain the captioned compound as a colorless oil (3.82 g, yield 68%).

Compound 5: $^1$H NMR (600 MHz/$CDCl_3$/TMS) δ 1.03 (3 H, s), 1.04 (3 H, s), 2.04 (1 H, t, J = 2.8 Hz), 2.38 (1 H, ddd, J = 16.5, 9.3, 2.8 Hz), 2.50 (1 H, dt, J = 16.5, 2.8 Hz), 2.63 (1 H, br.d, J = 2.8 Hz), 3.68 (1 H, d, J = 8.8 Hz), 3.77 (3 H, s), 3.83 (1 H, dt, J= 8.8 Hz), 6.83 (4 H, m). MS 248 (M+).
HRMS calcd. for $C_{15}H_{20}O_3$: 248.1413, found: 248.1408.

(Example 5) 4-(tert-Butyldimethylsilyl)oxy-6-(4-methoxyphenoxy)-5,5-dimethylhex-2-yne (Compound 6)

TBSOTf (1.5 eq) and 2,6-lutidine (3 eq) were added dropwise to a $CH_2Cl_2$ solution of Compound 5 (3.77 g, 15 mmols), and the mixture was stirred for 5 minutes at 0°C. The reaction mixture was extracted with EA. The EA layer was washed with water and brine, and dried over MaSO4. After filtration, the solvent was distilled off from the resulting filtrate. The residue was purified by silica gel column chromatography (EA:n-hexane = 1:12) to obtain the captioned compound as a colorless oil (4.45 g, yield 81%).

Compound 6: $^1$H NMR (600 MHz/$CDCl_3$/TMS) δ -0.01 (3 H, s), 0.15 (3 H, s), 0.88 (9 H, s), 1.00 (3 H, s), 1.04 (3 H, s), 1.98 (1 H, t, J = 2.8 Hz), 2.28 (1 H, ddd, J= 17.0, 4.9, 2.8 Hz), 2.57 (1 H, ddd, J= 17.0, 4.9, 2.8 Hz), 3.57 (1 H, d, J= 8.8 Hz), 3.74 (1 H, d, J= 8.8 Hz), 3.76 (1 H, s), 3.93 (1 H, t, J = 4.9 Hz), 6.81 (4 H, s).
MS 362 (M+), 347 (M-Me+), 305 (M-tBu+).
HRMS calcd. for $C_{21}H_{34}O_3Si$: 362.2278, found: 362.2285.

(Example 6) 3-(tert-Butyldimethylsilyl)oxy-2,2-dimethylhex-5-yn-1-ol (Compound 7)

Compound 6 (2.00 g, 5.5 mmols) was dissolved in a mixture of 48 mL acetonitrile and 12 mL water, and then the solution was cooled to 0°C. Then, CAN (2.4 eq) was added, and the resulting mixture was stirred for 15 minutes at 0°C. EA and brine were added for phase separation, whereafter the aqueous layer was extracted with EA. The organic layer was washed with a saturated solution of $NaHCO_3$ and brine, and dried over $MaSO_4$. After filtration, the solvent was distilled off from the resulting filtrate. The residue was purified by silica gel column chromatography (EA:n-hexane = 1:9) to obtain the captioned compound as a colorless oil (600 g, yield 42%).

Compound 7: $^1$H NMR (600 MHz/$CDCl_3$/TMS) δ 0.17 (3 H, s), 0.87 (3 H, s), 0.92 (9H, s), 1.03 (3 H, s), 2.04 (1H, t, J= 2.7 Hz), 2.34 (1 H, ddd, J= 17.6, 4.4, 2.7 Hz), 2.58 (1 H, ddd, J= 17.6, 6.0, 2.7 Hz), 3.35 (1 H, dd, J = 11.0, 6.0 Hz), 3.70 (1 H, m), 3.72 (1 H, dd, J = 6.0, 4.4 Hz). MS 199 (M-tBu+).
HRMS calcd. for $C_{10}H_{19}O_2Si$: 199.1154, found: 199.1156.

(Example 7) 3-(tert-Butyldimethylsilyl)oxy-2,2-dimethylhex-5-ynal (Compound 8)

4Å Molecular sieve (240 mg) was added to a $CH_2Cl_2$ solution of Compound 7 (633 g, 2.5 mmols), and PDC (1.02 g, 1.1 eq) was added at 0°C under an argon atmosphere. The resulting mixture was left to stand overnight at room temperature. The reaction mixture was purified by silica gel column chromatography (EA:n-hexane = 1:9) to recover Compound 7 (153 mg, 24%) and obtain the captioned compound as a colorless oil (230 mg, yield 37%).

Compound 8: $^1$H NMR (600 MHz/$CDCl_3$/TMS) δ 0.09 (3 H, s), 0.15 (3 H, s), 0.87 (9H, s), 1.08 (3 H, s), 1.09

(1H, t, J= 2.7 Hz), 2.02 (1 H, t, J= 2.8 Hz), 2.33 (1 H, ddd, J= 17.6, 4.9, 2.8 Hz), 2.45 (1 H, ddd, J= 17.6, 6.0, 2.8 Hz), 3.97 (1 H, t, J = 5.5 Hz), 9.67 (1 H,s).
MS 239 (M-Me+).
HRMS calcd. for $C_{13}H_{23}O_2Si$: 239.1468, found: 239.1472.

(Example 8) (3RS,5RS)-5-(tert-Butyldimethylsilyl)oxy-4,4-dimethyloct-1-en-7-yn-3-ol (Compound 9a: 1,3-anti) and (3RS, 5SR)-5-(tert-Butyldimethylsilyl)oxy-4,4-dimethyloct-1-en-7-yn-3-ol (Compound 9b: 1,3-syn)

To a toluene solution of Compound 8 (230 mg, 0.91 mmol), a vinylmagnesium bromide solution (0.57 mL, 1.1 eq) in THF was added dropwise at -78°C under an argon atmosphere, and the mixture was stirred for 60 minutes. A saturated solution of $NH_4Cl$ was added, and the mixture was extracted with EA. The EA layer was washed with brine, dried over $MaSO_4$, and filtered. Then, the solvent was distilled off from the resulting filtrate. The residue was purified by silica gel column chromatography (EA:n-hexane = 1:9) to obtain Compound 9a (53 mg, yield 20%) and Compound 9b (102 mg, yield 40%) as colorless oils.
Compound 9a: $^1$H NMR (600 MHz/$CDCl_3$/TMS) δ 0.15 (3 H, s), 0.20 (3 H, s), 0.82 (3 H, s), 0.93 (9 H, s), 0.98 (3 H, s), 2.04 (1 H, t, J = 2.7 Hz), 2.41 (1 H, ddd, J = 17.6, 4.9, 2.7 Hz), 2.66 (1 H, ddd, J = 17.6, 4.9, 2.7 Hz), 3.76 (1 H, t, J = 4.9 Hz), 3.86 (1 H, br.s), 4.31 (1 H, dt, J = 6.3, 1.1 Hz), 5.18 (1 H, ddd, J = 10.4, 1.9, 1.1 Hz), 5.28 (1 H, ddd J = 17.0, 1.9, 1.1 Hz), 5.84 (1 H, ddd, J = 17.0, 10.4, 6.3 Hz).
MS 282 (M+).
HRMS cald. for $C_{16}H_{30}O_2Si$: 282.2015, found: 282.2012.
Compound 9b: $^1$H NMR (600 MHz/$CDCl_3$/TMS) δ 0.12 (3 H, s), 0.17 (3 H, s), 0.85 (3 H, s), 0.92 (9 H, s), 0.93 (3 H, s), 2.04 (1 H, t, J = 2.8 Hz), 2.30 (1 H, ddd, J = 17.6, 4.4, 2.8 Hz), 2.34 (1 H, br.d, J = 3.8 Hz), 2.63 (1 H, ddd, J = 17.6, 6.0, 2.8 Hz), 3.82 (1 H, t, J = 4.4 Hz), 4.14 (1 H, m), 5.19 (1 H, ddd, J = 10.4, 1.7, 1.1 Hz), 5.27 (1 H, dt, J = 17.0, 1.7 Hz), 5.94 (1 H, ddd, J = 17.0, 10.4, 6.3 Hz). MS 282 (M+).
HRMS calcd. for $C_{16}H_{30}O_2Si$: 282.2015, found: 282.1994.

(Example 9) (3RS,5RS)-bis[(tert-Butyldimethylsilyl)oxy]-4,4-dimethyloct-1-en-7-yne (Compound 10a: 1,3-anti)

TBSOTf (1.5 eq) and 2,6-lutidine (3 eq) were added dropwise to a $CH_2Cl_2$ solution of Compound 9a (91 mg, 0.32 mmol), and the mixture was stirred for 60 minutes at 0°C. The reaction mixture was extracted with EA. The EA layer was washed with water and brine, and dried over $MaSO_4$. After filtration, the solvent was distilled off from the resulting filtrate. The residue was purified by silica gel column chromatography (EA:n-hexane = 1:12) to obtain the captioned compound as a colorless oil (126 mg (quantitative yield)).
Compound 10a: $^1$H NMR (600 MHz/$CDCl_3$/TMS) δ 0.00 (3 H, s), 0.08 (3 H, s), 0.15 (3 H, s), 0.82 (6 H, s), 0.86 (3 H, s), 0.89 (9 H, s), 0.91 (9 H, s), 1.97 (1 H, t, J = 3.1 Hz), 2.22 (1 H, ddd, J = 17.3, 5.8, 3.1 Hz), 2.56 (1 H, ddd, J = 17.3, 4.1, 3.1 Hz), 3.75 (1 H, dd, J = 5.8, 4.1 Hz), 4.01 (1 H, d, J = 8.0 Hz), 5.12 (1 H, d, J = 18.7 Hz), 5.13 (1 H, d, J = 10.4 Hz), 5.83 (1 H, ddd, J = 18.7, 10.4, 8.0 Hz).
MS 396 (M+), 381 (M-Me+), 339 (M-tBu+).
HRMS calcd. for $C_{22}H_{44}O_2Si_2$: 396.2880, found: 396.2910.

(Example 10) (3RS,5SR)-Bis[(tert-butyldimethylsilyl)oxy]-4,4-dimethyloct-1-en-7-yne (Compound 10b: 1,3-syn)

Compound 10b was synthesized from Compound 9b by the same procedure as described for Compound 10a.
Compound 10b: $^1$H NMR (600 MHz/$CDCl_3$/TMS) δ -0.01 (3 H, s), 0.04 (3 H, s), 0.09 (3 H, s), 0.17 (3 H, s), 0.76 (3 H, s), 0.86 (3 H, s), 0.90 (9 H, s), 0.92 (9 H, s), 1.96 (1 H, t, J = 2.7 Hz), 2.20 (1 H, ddd, J = 17.3, 6.3, 2.7 Hz), 2.60 (1 H, dt, J = 17.3, 2.7 Hz), 3.80 (1 H, dd, J = 6.3, 2.7 Hz), 4.03 (1 H, d, J = 7.1 Hz), 5.13 (1 H, d, J = 10.4 Hz), 5.14 (1 H, d, J = 17.3 Hz), 5.81 (1 H, ddd, J = 17.3, 10.4, 7.1 Hz).
MS 396 (M+), 339 (M-tBu+).
HRHS calcd. for $C_{22}H_{44}O_2Si_2$: 396.2880, found: 396.2889.

(Example 11) (5Z,7E)-(1S,3R)-2,2-Dimethyl-9,10-seco-5,7,10(19)-cholestatrien-1,3,25-triol (di-Me-(1α,3β), Compound 21) and (5Z,7E)-(1R,3S)-2,2-Dimethyl-9,10-seco-5,7,10(19)-cholestatrien-1,3,25-triol (di-Me-(1β,3α), Compound 22)

A toluene solution (3 mL) of Compound 10a (63 mg, 0.16 mmol), Compound 20 (prepared by the method described in J. Am. Chem. Soc., 114, 9836-45, 1992; 57 mg, 0.16 mmol) as the CD-ring portion, $Pd(Ph_3P)_4$ (55 mg, 0.3 eq) and triethylamine (2.5 mL) was stirred for 65 minutes at 125°C under an argon atmosphere. The reaction mixture was allowed to cool to room temperature, and was then diluted with ether. After filtration, the solvent was distilled off from the resulting filtrate. The residue was separated by silica gel thin-layer chromatography (EA:n-hexane = 1:3) to

obtain a coupling product as a colorless oil (71 mg, yield 66%).

1.0M TBAF (0.5 mL, 5 eq) was added to a THF solution of the resulting coupling product (71 mg, 0.11 mmol), and the mixture was stirred for 3 days at room temperature. Brine was added to the reaction mixture, and the system was extracted with EA. The EA layer was dried over $MaSO_4$, and filtered. Then, the solvent was distilled off from the resulting filtrate. The residue was separated by silica gel thin-layer chromatography (EA:n-hexane = 1:2) to obtain a 3-position-deprotected product (21 mg, yield 34%) and a 1,3-position-deprotected product (18 mg, yield 29%). The 1,3-position-deprotected product was subjected to recycling reversed-phase HPLC (acetonitrile:water = 85:15) to separate a dimethyl-1α,3β-compound (di=Me-(1α,3β), Compound 21) and a dimethyl-1β,3α-compound (di-Me-(1β,3α), Compound 22).

Compound 21 (di-Me-(1α,3β)): $^1$H NMR (600 MHz/$CDCl_3$/TMS) δ 0.54 (3 H, s), 0.93 (3 H, d, J = 6.6 Hz), 0.98 (3 H, s), 1.04 (3 H, s), 1.21 (6 H, s), 1.48 (1 H, d, J = 6.0 Hz), 1.49 (1 H, d, J= 5.8 Hz), 2.28 (1 H, d, J = 14.0, 6.6 Hz), 2.64 (1 H, dd, J = 14.0, 3.6 Hz), 2.81 (1 H, dd, J = 12.4, 4.4 Hz), 3.76 (1 H, dt, J = 3.8, 6.3 Hz), 3.99 (1 H, d, J = 5.5 Hz), 5.05 (1 H, t, J = 1.7 Hz), 5.31 (1 H, t, J = 1.7 Hz), 6.03 (1 H, d, J = 11.3 Hz), 6.36 (1 H, d, J = 11.3 Hz).
MS 444 (M+), 426 (M-$H_2O$+), 408 (M-2$H_2O$+), 393 (M-2$H_2O$-Me+), 390 (M-3$H_2O$+), 375 (M-3$H_2O$-Me+).
HRMS calcd. for $C_{29}H_{48}O_3$: 444.3604, found: 444.3600.

Compound 22 (di-Me-(1β,3α)): $^1$H NMR (600 MHz/$CDCl_3$/TMS) δ 0.54 (3 H, s), 0.93 (3 H, d, J = 6.6 Hz), 1.01 (3 H, s), 1.02 (3 H, s), 1.21 (6 H, s), 1.45 (1 H, d, J= 4.9 Hz), 1.49 (1 H, d, J = 6.0 Hz), 2.30 (1 H, d, J = 14.0, 7.4 Hz), 2.60 (1 H, dd, J = 14.0, 3.8 Hz), 2.82 (1 H, dd, J = 12.4, 4.4 Hz), 3.78 (1 H, ddd, J = 7.7, 6.0, 4.4 Hz), 3.96 (1 H, d, J = 5.2 Hz), 5.05 (1 H, m), 5.29 (1 H, dd, J = 1.9, 1.1 Hz), 6.02 (1 H, d, J = 11.3 Hz), 6.37 (1 H, d, J = 11.3 Hz).
MS 426 (M-$H_2O$+), 408 (M-2$H_2O$+), 390 (M-3$H_2O$+), 375 (M-3$H_2O$-Me+).
HRMS calcd. for $C_{29}H_{46}O_2$: 426.3498, found: 426.3498.

(Example 12) (5Z,7E)-(1S,3S)-2,2-Dimethyl-9,10-seco-5,7,10(19)-cholestatrien-1,3,25-triol (di-Me-(1α,3α), Compound 23) and (5Z,7E)-(1R,3R)-2,2-Dimethyl-9,10-seco-5,7,10(19)-cholestatrien-1,3,25-triol (di-Me-(1β,3β), Compound 24)

Compound 23 (1α,3α-compound, di-Me-(la,3a)) and Compound 24 (1β,3β-compound, di-Me-(1β,3β)) were synthesized from Compound 10b by the same procedure as that described in Example 11.

Compound 23 (di-Me-(1α,3α)): $^1$H NMR (600 MHz/$CDCl_3$/TMS) δ 0.53 (3 H, s), 0.93 (3 H, d, J = 6.6 Hz), 0.98 (3 H, s), 1.13 (3 H, s), 1.21 (6 H, s), 2.12 (1 H, d, J= 5.2 Hz), 2.40 (1 H, d, J = 14.3, 5.2 Hz), 2.66 (1 H, dd, J = 14.3, 2.2 Hz), 2.71 (1 H, d, J = 7.1 Hz), 2.84 (1 H, dd, J = 11.3, 2.8 Hz), 3.56 (1 H, ddd, J = 7.1, 5.2, 2.2 Hz), 3.80 (1 H, d, J = 4.9 Hz), 5.04 (1 H, d, J = 2.2 Hz), 5.26 (1 H, d, J = 2.2 Hz), 6.03 (1 H, d, J = 11.3 Hz), 6.43 (1 H, d, J = 11.3 Hz).
MS 444 (M+), 426 (M-$H_2O$+), 408 (M-2$H_2O$+), 393 (M-2$H_2O$-Me+), 390 (M-3$H_2O$+), 375 (M-3$H_2O$-Me+).
HRMS calcd. for $C_{29}H_{46}O_2$: 444.3604, found: 444.3611.

Compound 24 (di-Me-(1β,3β)): $^1$H NMR (600 MHz/$CDCl_3$/TMS) δ 0.55 (3 H, s), 0.94 (3 H, d, J = 6.6 Hz), 0.96 (3 H, s), 1.17 (3 H, s), 1.21 (6 H, s), 2.24 (1 H, d, J= 5.0 Hz), 2.39 (1 H, d, J = 14.6, 4.4 Hz), 2.71 (1 H, br.d, J = 14.0 Hz), 2.81 (1 H, d, J = 8.8 Hz), 2.84 (1 H, dd, J = 11.5, 3.3 Hz), 3.57 (1 H, m), 3.82 (1 H, d, J = 4.1 Hz), 5.07 (1 H, d, J = 2.2 Hz), 5.26 (1 H, d, J = 1.9 Hz), 6.07 (1 H, d, J = 11.3 Hz), 6.46 (1 H, d, J = 11.0 Hz).
MS 444 (M+), 426 (M-$H_2O$+), 408 (M-2$H_2O$+), 393 (M-2$H_2O$-Me+), 390 (M-3$H_2O$+), 375 (M-3$H_2O$-Me+).
HRMS calcd. for $C_{29}H_{46}O_2$: 444.3604, found: 444.3610.

(Test Example) Experiments on binding to bovine thymus vitamin D receptor (VDR)

The capability of the vitamin D derivatives of the present invention to bind to bovine thymus VDR was tested.

The vitamin D derivatives of the present invention used were the compounds synthesized in the above-described examples, i.e., (5Z,7E)-(1S,3R)-2,2-dimethyl-9,10-seco-5,7,10(19)-cholestatrien-1,3,25-triol (Compound 21), (5Z,7E)-(1R,3S)-2,2-dimethyl-9,10-seco-5,7,10(19)-cholestatrien-1,3,25-triol (Compound 22), (5Z,7E)-(1S,3S)-2,2-dimethyl-9,10-seco-5,7,10(19)-cholestatrien-1,3,25-triol (Compound 23), and (5Z,7E)-(1R,3R)-2,2-dimethyl-9,10-seco-5,7,10(19)-cholestatrien-1,3,25-triol (Compound 24).

In connection with each of Compounds 21 to 24 and 1α,25-dihydroxyvitamin $D_3$ (used as a standard), ethanol solutions at various concentrations were prepared in the following manner: In the case of 1α,25-dihydroxyvitamin $D_3$, serial dilutions were prepared at concentrations of 5 nanograms, 500 picograms, 250 picograms, 125 picograms, 63 picograms, 32 picograms, 16 picograms, 8 picograms, 4 picograms, 2 picograms, 1 picogram, 0.5 picogram, and 0.25 picogram as the amount of the compound contained in 50 microliters. In the case of the 1α,3β-compound and the 1α,3α-compound for the configurations of the substituents at the 1-position and the 3-position, serial dilutions were prepared at concentrations of 500 nanograms, 50 nanograms, 25 nanograms, 13 nanograms, 6.3 nanograms, 3.2 nanograms, 1.6 nanograms, 800 picograms, 400 picograms, 200 picograms, 20 picograms, and 2 picograms. In the case of the 1β,3β-compound and the 1β,3α-compound for the configurations of the substituents at the 1-position and the 3-position, serial dilutions were prepared at concentrations of 500 nanograms, 50 nanograms, 5 nanograms, 500 picograms, and

50 picograms.

Bovine thymus VDR was purchased from Yamasa Biochemical (Choshi, Chiba Prefecture, Japan; lot. 112831), and one ampoule thereof (ca. 25 mg) was dissolved in 55 ml of 0.05 M phosphate-0.5 M potassium buffer (pH 7.4).

The ethanol solution (50 $\mu$l) of each of Compounds 21 to 24 or 1$\alpha$,25-dihydroxyvitamin $D_3$ and the receptor solution (500 $\mu$l) were placed in a test tube, and preincubated for 1 hour at room temperature. Then, a [$^3$H]-1$\alpha$,25-dihydroxyvitamin $D_3$ solution (50 $\mu$l) was added at a final concentration of 0.1 nM, and the mixture was incubated overnight at 4°C. Dextran-coated charcoal was added to the reaction mixture, followed by mixing. Then, the mixture was left to stand for 30 minutes at 4°C, and centrifuged at 3,000 rpm for 10 minutes to separate the receptor-bound [$^3$H]-1$\alpha$,25-dihydroxyvitamin $D_3$ and the free [$^3$H]-1$\alpha$,25-dihydroxyvitamin $D_3$. The supernatant (500 $\mu$l) was mixed with ACS-II (9.5 ml) (Amersham, England) for radioactivity measurement.

The relative VDR-binding potency of each of Compounds 21 to 24 was calculated from the following equation, with the VDR-binding potency of 1$\alpha$,25-dihydroxyvitamin $D_3$ being taken as 100.

$$X = (y/x) \times 100$$

X: Relative VDR-binding potency of each of Compounds 21 to 24

y: The concentration of 1$\alpha$,25-dihydroxyvitamin $D_3$ needed to inhibit 50% of the binding of [$^3$H]-1$\alpha$,25-dihydroxyvitamin $D_3$ to VDR

x: The concentration of each of Compounds 21 to 24 needed to inhibit 50% of the binding of [$^3$H]-1$\alpha$,25-dihydroxyvitamin $D_3$ to VDR

The results are shown below.

(Table 1)

| Compound | Binding potency |
|---|---|
| Compound 21 (di-Me-(1α,3β)) | 3 |
| Compound 22 (di-Me-(1β,3α)) | 0.005 |
| Compound 23 (di-Me-(1α,3α)) | 0.06 |
| Compound 24 (di-Me-(1β,3β | <0.001 |

INDUSTRIAL APPLICABILITY

The vitamin D derivatives of the present invention, represented by the general formula (1), are novel compounds, and they are expected to be useful as medicines, such as calcium metabolism regulators.

## Claims

1. A vitamin D derivative represented by the following general formula (1):

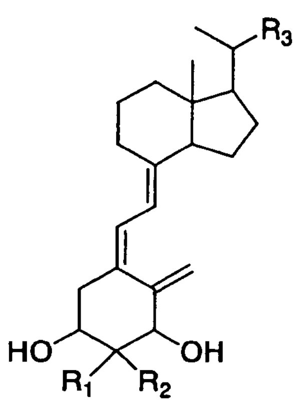

wherein

$R_1$ and $R_2$ may be the same or different, and each represents a straight chain or branched chain alkyl group optionally substituted by a hydroxyl group, and
$R_3$ represents a straight chain or branched chain alkyl group optionally substituted by a hydroxyl group.

**2.** The vitamin D derivative according to claim 1, wherein $R_1$ and $R_2$ may be the same or different, and each represents a straight chain or branched chain alkyl group having 1 to 6 carbon atoms and optionally substituted by a hydroxyl group, and $R_3$ represents a straight chain or branched chain alkyl group having 1 to 12 carbon atoms and substituted by a hydroxyl group.

**3.** The vitamin D derivative according to claim 1, wherein $R_1$ and $R_2$ may be the same or different, and each represents a straight chain or branched chain alkyl group having 1 to 3 carbon atoms and optionally substituted by a hydroxyl group, and $R_3$ represents a straight chain or branched chain alkyl group having 3 to 10 carbon atoms and substituted by a hydroxyl group.

**4.** The vitamin D derivative according to claim 1, wherein $R_1$ represents a methyl group, $R_2$ represents a methyl group, and $R_3$ represents a 4-hydroxy-4-methylpentyl group.

**5.** The vitamin D derivative according to any one of claims 1 to 4, wherein a steric configuration at a 20-position is the S-configuration.

**6.** The vitamin D derivative according to any one of claims 1 to 4, wherein a steric configuration at a 20-position is the R-configuration.

**7.** A pharmaceutical composition comprising the vitamin D derivative according to any one of claims 1 to 6.

**Patentansprüche**

**1.** Vitamin D-Derivat, das durch die folgende allgemeine Formel (1) dargestellt wird:

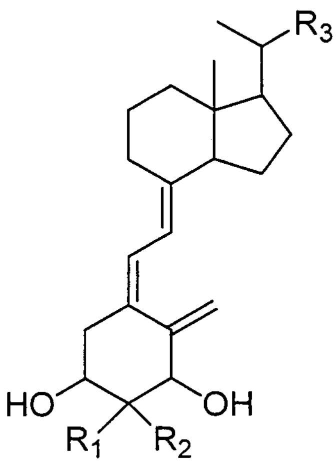

wobei

$R_1$ und $R_2$ gleich oder verschieden sein können, und jeder einen geradkettigen oder verzweigtkettigen Alkylrest darstellt, der gegebenenfalls mit einer Hydroxylgruppe substituiert ist, und $R_3$ einen geradkettigen oder verzweigtkettigen Alkylrest darstellt, der gegebenenfalls mit einer Hydroxylgruppe substituiert ist.

**2.** Vitamin D-Derivat gemäß Anspruch 1, wobei $R_1$ und $R_2$ gleich oder verschieden sein können, und jeder einen geradkettigen oder verzweigtkettigen Alkylrest mit 1 bis 6 Kohlenstoffatomen darstellt und der gegebenenfalls mit

einer Hydroxylgruppe substituiert ist, und $R_3$ einen geradkettigen oder verzweigtkettigen Alkylrest mit 1 bis 12 Kohlenstoffatomen darstellt und der gegebenenfalls mit einer Hydroxylgruppe substituiert ist.

**3.** Vitamin D-Derivat gemäß Anspruch 1, wobei $R_1$ und $R_2$ gleich oder verschieden sein können, und jeder einen geradkettigen oder verzweigtkettigen Alklyrest mit 1 bis 3 Kohlenstoffatomen darstellt und der gegebenenfalls mit einer Hydroxylgruppe substituiert ist, und $R_3$ einen geradkettigen oder verzweigtkettigen Alkylrest mit 3 bis 10 Kohlenstoffatomen darstellt und der gegebenenfalls mit einer Hydroxylgruppe substituiert ist.

**4.** Vitamin D-Derivat gemäß Anspruch 1, wobei $R_1$ eine Methylgruppe, $R_2$ eine Methylgruppe und $R_3$ eine 4-Hydroxy-4-methylpentylgruppe darstellt.

**5.** Vitamin D-Derivat gemäß einem der Ansprüche 1 bis 4, wobei eine sterische Konfiguration in der 20-Stellung die S-Konfiguration ist.

**6.** Vitamin D-Derivat gemäß einem der Ansprüche 1 bis 4, wobei eine sterische Konfiguration in der 20-Stellung die R-Konfiguration ist.

**7.** Arzneimittel umfassend das Vitamin D-Derivat gemäß einem der Ansprüche 1 bis 6.

**Revendications**

**1.** Dérivé de vitamine D représenté par la formule générale (1) suivante :

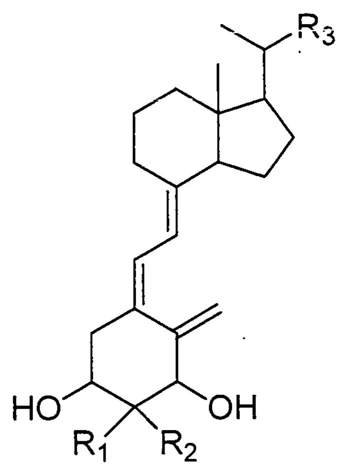

dans laquelle

$R_1$ et $R_2$ peuvent être identiques ou différents, et chacun représente un groupe alkyle à chaîne linéaire ou à chaîne ramifiée optionnellement substitué par un groupe hydroxyle, et
$R_3$ représente un groupe alkyle à chaîne linéaire ou à chaîne ramifiée optionnellement substitué par un groupe hydroxyle.

**2.** Dérivé de vitamine D selon la revendication 1, dans lequel $R_1$ et $R_2$ peuvent être identiques ou différents, et chacun représente un groupe alkyle à chaîne linéaire ou à chaîne ramifiée ayant 1 à 6 atomes de carbone et optionnellement substitué par un groupe hydroxyle, et $R_3$ représente un groupe alkyle à chaîne linéaire ou à chaîne ramifiée ayant 1 à 12 atomes de carbone et substitué par un groupe hydroxyle.

**3.** Dérivé de vitamine D selon la revendication 1, dans lequel $R_1$ et $R_2$ peuvent être identiques ou différents, et chacun représente un groupe alkyle à chaîne linéaire ou à chaîne ramifiée ayant 1 à 3 atomes de carbone et optionnellement substitué par un groupe hydroxyle, et $R_3$ représente un groupe alkyle à chaîne linéaire ou à chaîne ramifiée ayant 3 à 10 atomes de carbone et substitué par un groupe hydroxyle.

4. Dérivé de vitamine D selon la revendication 1, dans lequel $R_1$ représente un groupe méthyle, $R_2$ représente un groupe méthyle, et $R_3$ représente un groupe 4-hydroxy-4-méthylpentyle.

5. Dérivé de vitamine D selon l'une quelconque des revendications 1 à 4, dans lequel la configuration stérique à la position 20 est la configuration S.

6. Dérivé de vitamine D selon l'une quelconque des revendications 1 à 4, dans lequel la configuration stérique à la position 20 est la configuration R.

7. Composition pharmaceutique comprenant un dérivé de vitamine D selon l'une quelconque des revendications 1 à 6.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 51019752 A
- JP 2001393881 A


### Non-patent literature cited in the description


- **K. KONNO et al.** *Bioorg. Med. Chem. Lett.,* 1998, vol. 8, 151
- **T. FUJISHIMA et al.** *Bioorg. Med. Chem. Lett.,* 1998, vol. 8, 2145
- *J. Org. Chem.,* 1994, vol. 59 (25
- *J. Am. Chem. Soc.,* 1992, vol. 114, 9836-45